# EUROPÄISCHE PATENTANMELDUNG

(11) **EP 0 827 947 A1**
(43) Veröffentlichungstag der Anmeldung: **11.03.1998**
(21) Anmeldenummer: 97113719.5
(22) Anmeldetag: 08.08.1997
(51) Int. Cl.: C07C 67/08, C07C 69/80

(54) **Verfahren zur Herstellung von Carbonsäureestern und dafür geeignete Katalysatoren**

(30) Priorität: 04.09.1996 DE 19635769
(71) Anmelder: Degussa Aktiengesellschaft, 60387 Frankfurt am Main (DE)
(72) Erfinder: Wieland, Stefan, Dr., 63069 Offenbach (DE); Panster, Peter, Dr., 63517 Rodenbach (DE)

(57) **Zusammenfassung**

Die Erfindung betrifft ein Verfahren zur Herstellung von Carbonsäureestern, das gekennzeichnet ist durch die folgenden Schritte:
a) Ansetzen einer Reaktionsmischung bestehend aus einem Alkohol und einer Carbonsäure und/oder einem Carbonsäureanhydrid und/oder einem Carbonsäureester und oder einer teilweise veresterten Carbonsäure,
b) Erhitzen dieser Mischung in Gegenwart eines im festen im Reaktionsmedium unlöslichen Sulfonsäurengruppen-tragenden Polysiloxans unter intensiven Durchmischen auf die geeigneteReaktionstemperatur unter ständigem Abtrennen des sich bildenden Reaktionswassers, wobei das Polysiloxan bevorzugt in einer mit einer löslichen Aluminium-, Titan- oder Zirkoniumverbindung nachbehandelten Modifikation eingesetzt wird, und die kugelförmigen Teilchen einen Durchmesser von 0,01 bis 3 mm, eine spezifische Oberfläche von 0,1 bis 1200 m²/g, ein spezifisches Porenvolumen von 0,01 bis 6,0 ml/g und eine Schüttdichte von 50 bis 1000 g/l besitzen.

## Beschreibung

Die Erfindung betrifft ein Verfahren zu Herstellung von Carbonsäurseestern und dafür geeignete Katalysatoren. Sauer katalysierte Ver- und Umesterungen werden nach dem Stand der Technik z. B. Schwefelsäure, Salzsäure oder auch löslichen organischen Säuren durchgeführt. Homogene Säurekatalysatoren zeichnen sich durch eine besonders gute Aktivität aus. Nachteil ihres Einsatzes ist aber die schlechte Abtrennbarkeit vom Reaktionsprodukt und insbesondere die Notwendigkeit einer ggf. destillativen Aufreinigung des Veresterungsproduktes. Hierbei kann es zu unerwünschten Verfärbungen des Produktes bzw. zum Verlust von Produkt kommen, insbesondere wenn es sich im höher molekulare Reaktionsprodukte handelt. Es ist ebenfalls bekannt, die Esterbildung in Gegenwart fester saurer Katalysatoren vorzunehmen.

Derartige Verfahren werden in der EP-B-0192035 für Kationenaustauscherharze und in der US 5502240 für Titanhaltige Zeolithe als Katalysatoren beschrieben. Gemäß dieser Patentschrift erfolgt die Herstellung von Phthalsäureestern bei Temperaturen von 210 bis 230 °C.

Gegenstand der Erfindung ist ein Verfahren zur Herstellung von Carbonsäureestern, gekennzeichnet durch die folgenden Schritte:
a) Ansetzen einer Reaktionsmischung bestehend aus einem Alkohol und einer Carbonsäure und/oder einem Carbonsäureanhydrid und/oder einem Carbonsäureester und oder einer teilweise veresterten Carbonsäure,
b) Erhitzen dieser Mischung in Gegenwart eines im festen im Reaktionsmedium unsöslichen Sulfonsäurengruppen-tragenden Polysiloxans unter intensiven Durchmischen auf die geeignete Reaktionstemperatur unter ständigem Abtrennen des sich bildenden Reaktionswassers und
c) Isolieren des gewünschten Esters nach der Umsetzung z.B. durch Destillation nach der Abtrennung des Katalysators.

Als Katalysatoren werden erfindungsgemäß solche eingesetzt, wie sie in den Patentschriften DE 3226093 (^ EP 0098946, US 4552700), DE 3518881 und DE 4223539 (^ US 5354831) beschrieben werden.

Die verwendeten Organopolysiloxane sind beispielsweise dadurch gekennzeichnet, daß sie gleiche oder verschiedene Einheiten der allgemeinen Formel

(O_{3/2}Si--R¹--SO₃⁻)ₓM *⁺

enthalten, in der R¹ für eine lineare oder verzweigte Alkylengruppe mit 1 bis 12 C-Atomen, eine Cycloalkylengruppe mit 5 bis 8 C-Atomen oder für Einheiten 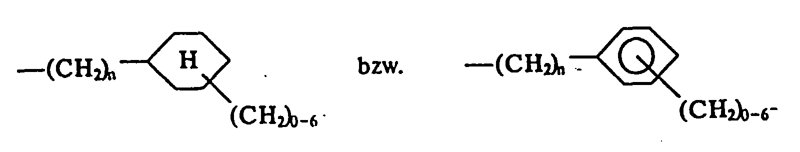 steht, in denen n eine Zahl von 1 bis 6 sein kann und die Zahl der Schwefel-ständigen Methylgruppen angibt, x in Abhängigkeit von M eine Zahl von 1 - 4 bedeutet, und M für Wasserstoff oder ein 1- bis 4wertiges Metallion steht und die freien Valenzen der Sauerstoffatome durch Siliciumatome weitere Gruppen der Formel (1)und/oder durch vernetzende Brückenglieder
SiO_{4/2} oder R'SiO_{3/2} oder R'₂SiO_{2/2} bzw.
TiO_{4/2} oder R'TiO_{3/2} oder R'₂TiO_{2/2} bzw.
AlO_{3/2}
abgesättigt sind.

Weitere Einzelheiten sind den zitierten Patentschriften zu entnehmen.

Diese enthalten auch Angaben über die bevorzugt eingesetzten kugelförmigen Teilchen dieser Zusammensetzung, die einen Durchmesser von von 0,01 bis 3mm, eine spezifische Oberfläche von 0,1 bis 1200 m²/g und eine Schüttdichte von 50 bis 1000 g/l besitzen.

In einer bevorzugten Ausführungsform setzt man mit Aluminium, Titan- oder Zirkonverbindungen modifizierte Katalysatoren dieses Typs ein. Derartig modifizierte Katalysatoren, die ebenfalls beansprucht werden, erhält man, indem man die oben genannten Sulfongruppen-haltigen Polysiloxane unter hydrothermalen Bedingungen mit der Lösung einer oder mehrer dieser Elemente behandelt.

Im allgemeinen liegen die phys.-chem. Parameter , wie Durchmesser, spez. Oberfläche, Porenvolumen und Schüttdichte dann in den oben genannten Bereichen.

Die löslichen Aluminium-, Titan- und/oder Zirkoniumverbindungen werden bevorzugt in Form von wäßrigen, alkoholischen, insbesondere ethanolischen oder methanolischen, Lösungen eingesetzt. Diese enthalten dann vorzugsweise Hydroxykomplexe, Acetylacetonatkomplexe, Alkoxiverbindungen oder auch sonstige komplexgebundene Formen.

Die Modifizierung erfolgt bei 80 bis 200 °C, bevorzugt im Temperaturbereich von 100 bis 180 °C bei einem Druck, der der Summe der Partialdrücke der einzelnen Komponenten entspricht.

Nach der Umsetzung wird der Feststoff abgetrennt, gegebenfalls gewaschen und dann getrocknet.

Die Menge der auf die zu modifizierenden Polysiloxane aufgebrachten Al-, Ti- und/oder Zr-Verbindungen ist weitgehend unabhänig von deren Konzentration in den zum Modifizieren eingesetzten Lösungen.

Im allgemeinen wählt man diese Konzentration ca. 10 % höher als die Konzentration, die man auf dem Feststoff wiederfinden will.

Es hat sich gezeigt, daß der Einbau von Al-Konzentrationen im Bereich von 0,1 ‰ bis 1 %, der von Ti und Zr-Konzentrationen im Bereich von 0,1 ‰ bis 5 % liegt, bezogen auf die Gesamtmenge des Feststoffs.

Diese Mengen sind auch durch Waschprozesse nicht zu beseitigen.

Diese in oxidischer Form vorliegenden Verbindungen können von dem ursprünglich in dem Polysiloxan gegebenenfalls vorhandenen Al-, Ti oder Zr-Ionen durch analytische Methoden aufgrund ihrer nicht homogenen Verteilung über die gesamte Oberfläche des Katalysators unterschieden werden.

Das erfindungsgemäße Verfahren eignet sich insbesondere für die Herstellung von Phthalaten, wie z. B.

| |
|---|
| Dimethylphthalat |
| Diethylphthalat |
| Dibutylphthalat |
| Diisobutylphthalat |
| Butylbenzylphthalat |
| Diisopentylphthalat |
| Diheptylphthalat |
| Di-2-ethylhexylphthalat |
| Diisooctylphthalat |
| Di-*n*-octylphthalat |
| Di(hexyl-octyl-decyl)phthalat |
| Di(octyl-nonyl-decyl) phthalat |
| Di(hectyl-nonyl) phthalat |
| Di(heptyl-nonyl-undecyl) phthalat |
| Di(nonyl-decyl-undecyl) phthalat |
| Dinonylphthalat |
| Diisononylphthalat |
| Di(3,5,5-trimethylhexyl)phthalat |
| Diisodecylphthalat |
| Diundecylphthatalat |
| Diisoundecylphthalat |
| Diisotridecylphthalat |
| Di(methoxyethyl)phthalat |
| Di(butoxyethyl)phthalat |

bevorzugt Di-2-ethylhexylphthalat, Di-isooctylphthalat und Di-*n*-octylphthatalat. Hierbei setzt man insbesondere Phthalsäureanhydrid und den entsprechenden Alkohol ein.

Die Herstellung der Phthalate erfolgt bei Temperaturen von 100 bis 220°C, bevorzugt im Bereich von 130 bis 170°C. Neben der Herstellung von Phthalaten eignet sich das Verfahren unter Verwendung der oben beschriebenen Katalysatoren auch für die Veresterung freier Fettsäuren z. B. zu den Methylestern oder zu Estern mit höherem molekularem Alkoholanteil (z. B. die Veresterung von Fettsäuren mit Fettalkoholen). Das erfindungsgemäße Verfahren ist ebenfalls von großer Bedeutung für die Umesterung von Fettsäureestern und insbesondere für die Umesterung von Triglyceriden zu den Fettsäuremethylestern. Der umzusetzende Alkohol (bei einwertigen Alkoholen) liegt bevorzugt zumindest in einem molaren Verhältnis von 1 : 1 bis 1 : 1,5 bezogen auf die zu veresternden Säuregruppen vor. Als Alkohole setzt man vor allem solche mit einer Kettenlänge von C₁ bis C₂₀ , insbesondere C₄ bis C₁₂, geradkettig oder verzweigt, ein. Darunter sind bei entsprechender Kohenstoffzahl auch zyklische Alkohole mit einem gesättigten oder ungesättigten Ring zu zählen. Die Vorteile des erfindungsgemäßen Verfahrens liegen vor allem darin, daß die Reaktionstemperaturen gegenüber dem Stand der Technik deutlich abgesenkt werden können, ohne daß die Selektivität der Reaktion darunter leidet.

Wesentlich für das Erreichen dieses Ergebnisses ist eine intensive Durchmischung der Reaktionsmischung, in der der Katalysator suspendiert vorliegt.

Diese Durchmischung erfolgt auf bekannte Weise.

Gleichzeitig hat es sich als wichtig herausgestellt, das entstehende Reaktionswasser sofort abzutrennen. Dies entsteht durch Abdestillieren gegebenfalls unter reduziertem Druck oder unterstützt durch einen durch das Reaktionsgefäß geleiteten Gasstrom. Die Reaktionstemperatur kann über oder unter der Siedetemperatur des Alkohols liegen oder gleich der Siedetemperatur des Alkohols sein. Gerade im Fall des Einsatzes von hochsiedenden Alkoholen hat sich jedoch gezeigt, daß das erfindungsgemäße Verfahren dann besonders gute Ergebnisse liefert (niedrige Restsäurezahlen, niedrige Farbwerte), wenn die Reaktionstemperatur deutlich unter der Siedetemperatur des Alkohols gewählt wird, aber so hoch gewählt wird, daß eine sichere quantitative Entfernung des Reaktionswassers - unterstützt durch ein zusätzliches Durchleiten von Stickstoff durch das Reaktionsgefäß - gewährleistet ist. Die Veresterung kann stufenweíse in Form einer Reaktionskaskade durchgeführt werden. Teilweise verestertes Material wird so in einer folgenden Reaktionskaskade weiter verestert und letzendlich in einer letzten Reaktionskaskade solange umgesetzt bis die gewünschte Restsäurezahl erreicht wird.Der überschüssige Alkohol kann in der letzten Reaktionskaskade destillativ entfernt werden.

Als besonders günstig (zur Unterdrückung der Nebenproduktbildung aus dem Alkohol)hat es sich herausgestellt, wenn die Reaktionstemperatur bei der Veresterung unter der Siedetemperatur des Alkohols liegt und der Alkohol erst nach der Abtrennung des Rohproduktgemisches vom Katalysator destillativ entfernt wird. Das Verfahren läßt sich vorteilhaft z. B. in einer Rührkesselkaskade oder in Form hintereinandergeschalteter mehrerer Festbettreaktoren mit jeweils zwischengeschalteten Destillationsstufen zur Entfernung des Reaktionswassers durchführen. Die Auswirkungen nachträglichen Oberflächenmodifizierung der Katalysatoren sind insbesondere in der Erhöhung der Selektivität bei katalytischen Umsetzungen zu sehen. Durch Einbau von Aluminateinheiten mittels dieser nachträglichen Modifizierung, im Bereich von 0,1 ‰ bis 1 % wird die Hydrolyseanfälligkeit der Polysiloxanmatrix unter hydrothermalen Reaktionsbedingungen herabgesetzt. Durch Aufbringen von Titanoxid- bzw. Zirkonoxideinheiten wird die Selektivität der Katalysatoren dahingehend verbessert, daß die Nebenproduktbildung aus dem eingesetzten Alkohol (Olefinbildung, Etherbildung) reduziert ist. Die Vorteile des erfindungsgemäßen Verfahrens und der erfindungsgemäßen Katalysatoren zeigen sich z. B. bei der Veresterung von Phthalsäureanhydrid mit 1-Octanol oder 2-Ethylhexanol. Wird in diesem Fall das erfindungsgemäße Verfahren bei einer Reaktionstemperatur von 150 bis max. 160°C praktiziert und ein titanmodifizierter Katalysator (Titangehalt 5 Gew.%) eingesetzt, so erhält man nach Abtrennung des Rohproduktgemisches vom Katalysator und destillativer Abtrennung des überschüssigen Alkohols eine Restsäurezahl des Diesters von <0,1 mg KOH/g, und die abdestillierte 2-Ethylhexanol-phase enthält Nebenprodukte wie Olefine und Ether in einem Gewichtsanteil von <0,5 Gew.%.

Die Veresterung von freier Rindertalgfettsäure mit 1-Octanol erfolgt bei einer Reaktionstemperatur zwischen 150 und 180°C. Das erhaltene Reaktionsprodukt hat eine Restsäurezahl von <0,2 mg KOH/g.

## Patentansprüche

1. Verfahren zur Herstellung von Carbonsäureestern, gekennzeichnet durch die folgenden Schritte:
a)Ansetzen einer Reaktionsmischung bestehend aus einem Alkohol und einer Carbonsäure und/oder einem Carbonsäureanhydrid und/oder einem Carbonsäureester und oder einer teilweise veresterten Carbonsäure,
b)Erhitzen dieser Mischung in Gegenwart eines im festen im Reaktionsmedium unlöslichen Sulfonsäurengruppen-tragenden Polysiloxans unter intensiven Durchmischen auf die geeignete Reaktionstemperatur unter ständigem Abtrennen des sich bildenden Reaktionswassers und
c)Isolieren des gewünschten Esters nach der Umsetzung.

2. Verfahren gemäß Anspruch 1,
dadurch gekennzeichnet, daß man ein mit einer löslichen Titan-,Zirkon- und/oder Aluminiumverbindung bei 80 bis 200 °C umgesetztes Sulfonsäuregruppen tragendes Polysiloxan einsetzt.

3. Verfahren gemäß den Ansprüchen 1 und 2,
dadurch gekennzeichnet, daß man bei einer Reaktionstemperatur von 100 bis 220 °C arbeitet.

4. Verfahren gemäß den Ansprüchen 1 bis 3,
dadurch gekennzeichnet, daß man zur Entfernung des Reaktionswassers gegebenenfalls zusätzlich einen Inertgasstrom durchleitet.

5. Verfahren gemäß der Ansprüche 1 bis 4,
dadurch gekennzeichnet, daß man den Katalysator in einer Menge von bis zu 15 Gew.-% einsetzt, bezogen auf die Menge der Reaktionsmischung.

6. Verfahren gemäß der Ansprüche 1 bis 5,
dadurch gekennzeichnet, daß man einen Alkohol mit einer Kettenlänge von C₁ bis C₂₀ einsetzt.

7. Verfahren gemäß Anspruch 6,
dadurch gekennzeichnet, daß man einen Alkohol mit einer Kettenlänge von C₄ bis C₁₂ einsetzt.

8. Verfahren gemäß den Ansprüchen 1 bis 7,
dadurch gekennzeichnet, daß man freie Fettsäuren verestert.

9. Verfahren gemäß den Ansprüchen 1 bis 7,
dadurch gekennzeichnet, daß man Fettsäuren mit Fettalkoholen verestert.

10. Verfahren gemäß den Ansprüchen 1 bis 7,
dadurch gekennzeichnet, daß man als zu veresternde Säurekomponente Phthalsäureanhydrid, Phthalsäure und/oder Phthalsäuremonoester einsetzt.

11. Verfahren gemäß den Ansprüchen 1 bis 10,
dadurch gekennzeichnet, daß das molare Verhältnis von im Reaktionsgemisch vorliegenden Alkohol und zu veresternden Säuregruppen zwischen 1 : 1 und 1 : 1,5 liegt.

12. Verfahren gemäß den Ansprüchen 10 und 11,
dadurch gekennzeichnet, daß man Isooctyl- oder 1-Octylalkohol zu Veresterung einsetzt.

13. Verfahren gemäß den Ansprüchen 10 bis 12,
dadurch gekennzeichnet, daß man die Veresterung bei einer Temperatur von 100 bis 180 °C durchführt.

14. Sulfonsäuregruppen-haltiges Polysiloxan, dadurch gekennzeichnet, daß die Oberfläche durch 0,1 ‰ bis 1 % oxidischer Aluminiumverbindungen modifiziert vorliegt (bezogen auf die Gesamtmenge des Feststoffs).

15. Sulfonsäuregruppen-haltiges Polysiloxan, dadurch gekennzeichnet, daß die Oberfläche durch 0,1 ‰ bis 5 % oxidischer Titan oder Zirkonverbindungen modifiziert vorliegt (bezogen auf die Gesamtmenge des Feststoffs).

16. Polysiloxan gemäß den Ansprüchen 14, 15,
dadurch gekennzeichnet, daß die kugelförmigen Teilchen einen Durchmesser von 0,01 bis 3 mm, eine spezifisches Porenvolumen von 0,01 bis 6,0 ml/g und eine Schüttdichte von 50 bis 1000 g/l besitzen.

17. Verfahren zur Herstellung von an der Oberfläche modifizierten Sulfonsäuregruppen-haltigen Polysiloxanen, dadurch gekennzeichnet, daß man das genannte feste Polysiloxan in alkoholischem, neutralem, alkalischem oder saurem Medium bei Temperaturen von 80 bis 200 °C mit einer gelösten Titan-, Zirkon- und/oder Aluminiumverbindung umsetzt und das modifizierte Polysiloxan abtrennt, gegebenenfalls wäscht und dann trocknet.
